Europäisches Patentamt

(19) European Patent Office

Office européen des brevets

(11) Publication number: **0 108 736**
**A1**

(12) # EUROPEAN PATENT APPLICATION

(21) Application number: **83850294.6**

(22) Date of filing: **03.11.83**

(51) Int. Cl.³: **A 61 F 5/44**

(30) Priority: **04.11.82 SE 8206266**

(43) Date of publication of application:
**16.05.84 Bulletin 84/20**

(84) Designated Contracting States:
**CH DE FR GB LI**

(71) Applicant: **Industridesign Konsult Hans Tollin**
**Karl Johansgatan 62, 6tr.,**
**S-414 55 Göteborg(SE)**

(71) Applicant: **Industridesign Konsult Kenneth Österlin**
**Karl Johansgatan 62, 6tr.,**
**S-414 55 Göteborg(SE)**

(72) Inventor: **Tollin, Hans**
**Godhemsgatan 40A**
**S-414 68 Göteborg(SE)**

(72) Inventor: **Österlin, Kenneth**
**Nordhemsgatan 74**
**S-413 09 Göteborg(SE)**

(74) Representative: **Roth, Ernst Adolf Michael et al,**
**GÖTEBORGS PATENTBYRA AB Box 5005**
**S-402 21 Göteborg(SE)**

(54) A connecting tube for collector device for incontinent persons.

(57) A connecting tube for a collector device intended for continent persons, especially suffering from urine incontinence and which device is of the type which incorporates a urine collecting receptacle (9) intended to be worn with a suspending device on the thigh or the lower leg and where the tube (20; 10, 12) connected to the receptacle is intended to be interconnected with a uridome, a catheter or the like, whereby the tube is a broad, initially mainly flat tube preferably made from a soft foil, in order to eliminate the risk for leakage in the joints between receptacle and tube.

## FIG 3

## FIG 2

Croydon Printing Company Ltd

A connecting tube for collector device for incontinent persons

Background of the invention

The present invention relates to a connecting tube at collector device for incontinent persons, especially for urine and of the type incorporating a urine collecting receptacle, intended to be worn with a suspending device on the thigh or the lower leg and where the tube, which is attached to the receptacle is intended to be interconnected with a uridome, a catheter or the like.

Urine collecting devices of this type are earlier known in different embodiments and makes, which in many cases operate rather satisfactorily. The connecting tube, which is intended to interconnect the receptacle and the catheter however involves problems that are hard to master and which concern discomfort to the user, low flow rate and risk for leakage particularly at the joint between receptacle and tube, at the same as the flow in the tube can be obstructed and even completely blocked due to the tube being bent or being jammed. Due to the small tube dimension liquid can easily remain standing in the tube, as air can not come down from above, and this may result in a "back-suck". Conventional round plastic tubes finally are rather expensive.

The purpose and most essential features of the invention

The purpose of the invention is to provide a connecting tube at collector devices of the present type, by means of which the abovementioned drawbacks have been largely eliminated and this has been achieved by the features defined in the characterizing part of the appended claim 1.

Brief description of the drawings

The invention will hereinafter be further described with reference to an embodiment shown in the accompanying drawings.

Figure 1 shows in a schematic over-all view an exampel of the connection between an urine collecting bag and uridome or catheter, with a conventional tube and ditto tube joints.

Figure 2 shows in larger scale a urine bag equipped with tube joints according to the invention, and

Figure 3 shows an enlargement of a portion of a conneting tube according to the invention.

## Description of the embodiment

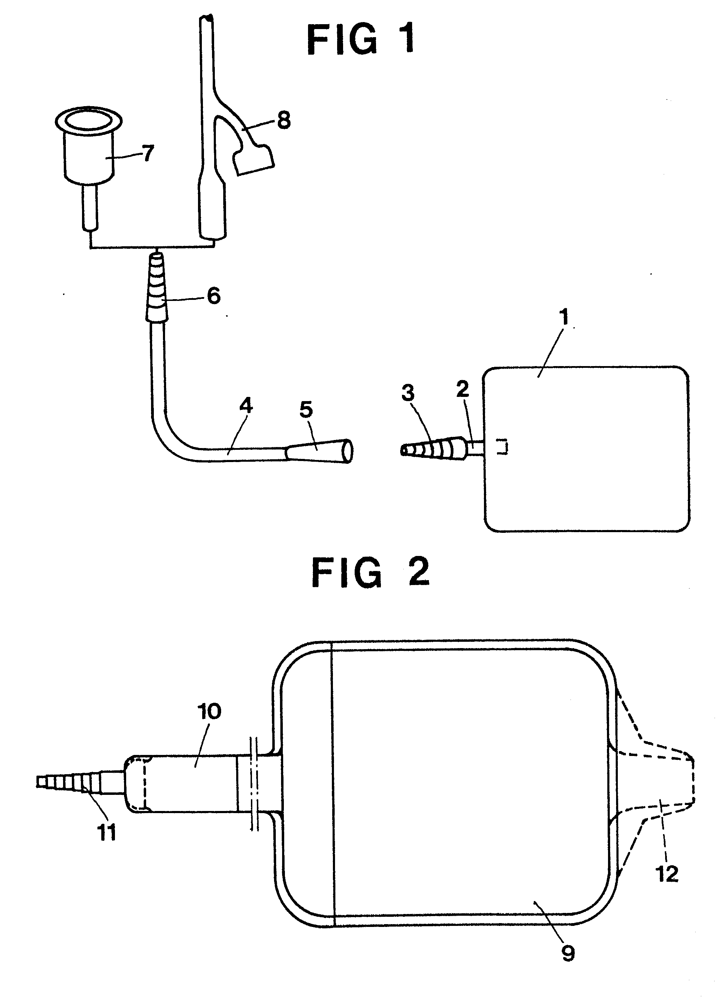

In figure 1 is schematically shown a urine collector system for incontinent persons comprising a urine collecting bag 1 provided with an inlet tube joint designed as a short length of a conventional round, rather rigid but pliable plastic tube 2, which on its end remote from the bag has a connection piece 3, mating into a corresponding receiving piece 5 attached to a tube 4. The tube 4 is likewise of conventional type and consists of a rather stiff plastic tube having a round crosssection and at its opposite end it is provided with another connecting piece 6, for interconnection with an uridome 7 or a catheter 8. As seen, the tube can be a member quite separate from the receptacle, which gives possibility of extended re-use. The mots common embodiment however has a tube which is fixedly attached to the bag and which at its free end is equipped with an attachment piece corresponding to the part 6. At the joint between the urine collecting bag 1 and the inlet tube 2 there is however difficulties in obtaining a leak-proof connection between the two foil surfaces of the bag which engage each other and the round tube socket disposed therebetween. The rather thin, conventional tube has no capacity of swallowing big flow volumes which gives rise to the risk for overflow. There is also risk that the tube 4 during use, when worn hidden beneath the clothes, can be bent so much that the urine flow therethrough will become slower or even become stopped, which means overflow in catheter or uridome with following discomfort and inconvenience.

Figur 2 shows in bigger scale a urine collecting bag 9, of the type having an inlet joint 10 equipped with an connecting member 11 and an intimated outlet portion 12, which can be opened for emptying the contents of the bag and thereupon again be leak-proff closed.

The inlet joint 10 as well as the outlet portion 12 are in accordance with the invention designed as tube material, which differs from that shown in figure 1 in that it is a non-round, broad material, which means that the problem as to leakproofness between tube and bag 9 has been removed.

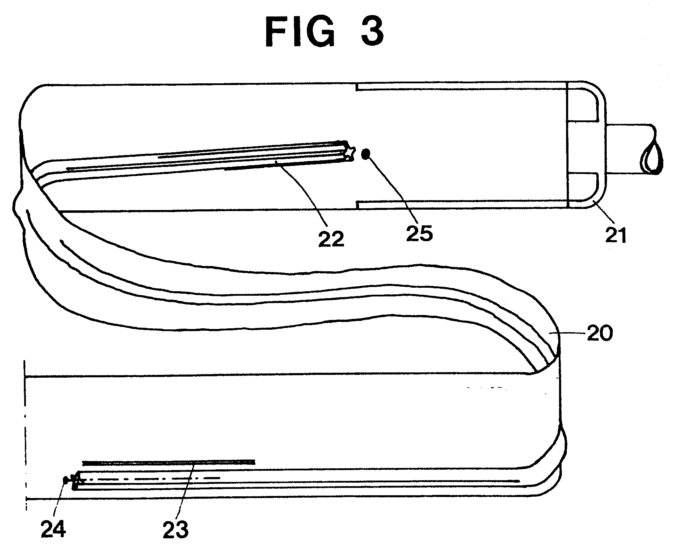

Figure 3 shows a connecting tube 20 according to the invention, which unlike conventional round tubes eliminate most of the sealing problems between bag and tube, at the same time as a better urine flow is obtained. The material cost is furthermore lower than that for a round tube.

This tube 20 preferably consists of two foils, which are welded together in the same manner as the bag, or it can alternatively be e.g. an extruded foil tube. This tube has itself a number of good advantages, whereby it e.g. is more flexible than the round, stiff tube, and it can better than this follow the body of the user. It is furthermore suited for having a nonreturn valve designed as foil tabs built into itself. As the tube is broad its area is will be big and urine will not remain standing in the tube. In the embodiment shown the tube has at one of its ends a connecting member 21, which can be welded to the tube. In order to eliminate the risk for the tube being blocked when bent or folded, it is preferably provided with a thin, pliable thread or profile 22, having a nonregular shape, and an ability of all the time keeping the passage through the tube open and prevent said foldings from blocking the passage or that the foils adhere against each other thus that the flow is obstructed. In order to ascertain that the profile 22 will not move in the tube 20 and in which case it could travel down into the bag or up into a nonreturn valve fitted at the opposite end of the tube, it is suitable to arrest the profile in its position, which can be accomplished e.g. by providing the tube with one or more continuous or dot-shaped welds 23, 24 and 25 resp. The profile can however also be formed directly in the material of the tube.

The invention is not limited to the embodiment shown but modifications are possible within the scope of the appended claims. The invention has hereinbefore been described in connection with collector bags for urine incontinent persons but it is to be understood that the same solutions can be applied also on stoma collector bags and the like.

0108736

## CLAIMS

1. A connecting tube for collector device for incontinent persons, especially for urine and of the type incorporating a urine collecting receptacle (1,9) intended to be worn with a suspending device on the thigh or the lower leg and where the tube (4,20;2,10,12), which is attached to the receptacle is intended to be interconnected with a uridome, catheter or the like,
characterized in that the connecting tube (20,10,12) is a broad, initially mainly flat tube.

2. A connecting tube according to claim 1,
characterized in that the tube is made from a soft foil.

3. A connecting tube according to claim 1 or 2,
characterized in that a thin pliable profile (22), which has an irregular crosssection, is inserted into the tube (20) in order to maintain the passage therethrough in open position.

4. A connecting tube according to claim 3,
characterized in that the profile is prevented from movement in the tube.

5. A connecting tube according to claim 1 or 2,
characterized in that the tube is provided with an internal ridge or flange formed in the material of the tube and acting in order to maintain the passage therethrough in open position.

0108736

1/2

FIG 1

FIG 2

# FIG 3

22 25 21

20

24 23

European Patent
Office

**EUROPEAN SEARCH REPORT**

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl. ³) |
| X | FR-A-2 350 828  (HOLLISTER)<br>* Whole document * | 1,2 | A 61 F    5/44 |
| A | EP-A-0 003 470  (ORSING)<br>* Whole document * | 3,4,5 | |
| | | | **TECHNICAL FIELDS SEARCHED (Int. Cl. ³)**<br><br>A 61 F<br>A 61 C |

The present search report has been drawn up for all claims

| Place of search<br>THE HAGUE | Date of completion of the search<br>02-02-1984 | Examiner<br>LOWE D. |
|---|---|---|